# EUROPEAN PATENT SPECIFICATION

(11) **EP 2 410 913 B1**
(45) Date of publication and mention of the grant of the patent: **03.09.2014**
(21) Application number: 10712124.6
(22) Date of filing: 22.03.2010
(51) Int. Cl.: A61B 5/117, G06K 9/00, C25D 13/04, C09D 5/44

(54) **METHOD OF VISUALISING A (FINGER)PRINT ON A SURFACE**
VERFAHREN ZUR VISUALISIERUNG EINES (FINGER-)ABDRUCKS AUF EINER OBERFLÄCHE
PROCÉDÉ DE VISUALISATION D'UNE EMPREINTE (DIGITALE) SUR UNE SURFACE

(30) Priority: 24.03.2009 GB 0904946
(43) Date of publication of application: 01.02.2012
(73) Proprietor: University Of Leicester, Leicester Leicestershire LE1 7RH (GB)
(72) Inventor: HILLMAN, Arthur, Leicester Leicestershire LE1 7RH (GB)
(74) Representative: Hutter, Anton
(86) International application number: PCT/GB2010/050479
(87) International publication number: WO 2010/109222

(56) References cited:
- WO-A1-2006/079599
- WO-A1-2008/044494
- FR-A1- 2 634 487
- GB-A- 2 449 264
- US-B1- 7 182 817

## Description

The present invention relates to a method of visualizing a print on a surface.

The uniqueness of an individual's fingerprints has long been recognized as a means of identification, whether for purposes of authentication, for establishing the identities of victims of natural disasters or for criminal investigations. Indeed, the notion of a fingerprint as a unique identifier is so deeply ingrained that the term "fingerprinting" is frequently used colloquially in the context of other means of identification, e.g. in DNA fingerprinting of people or the fingerprint region of an infra-red spectrum.

Since the most natural means of exchange of material between an individual and an object is associated with the object being held, fingerprint recognition remains one of the most potent and incontrovertible means of demonstrating the presence of an individual at the scene of a crime or other incident. As a consequence, any means of extending the capability to acquire fingerprint information is of considerable significance and utility.

Fingerprints are created by the perspiration secreted from the sweat pores found along the friction skin ridges present on the fingertips; these ridges extend to the rest of the hand and to the feet, but the majority of useful information derives from the fingertips. Some of this secretion is inevitably transferred to any object coming into contact with the fingertips (Lee HC, Gaensslen RE. Advances in Fingerprinting Technology. 2nd Ed. London: CRC Press, 2001).

While some circumstances (including transfer of paint, blood or foodstuffs) may lead to visible fingerprint images, the majority of transfers lead to fingerprint images that are not directly visible. Over the past century there have been innumerable methods and reagents explored for the development of such latent fingerprints, including metallic and other powders, cyanoacrylate ("superglue") fuming and fluorescent dyes (see Lee HC et al).

The common feature is that these reagents interact in some way - physically adhere, chemically react or are optically perturbed - with one or more constituents of the fingerprint deposit. Decoration of the fingerprint deposit by the reagent then generates visual contrast between the ridge detail and the substrate.

Difficulties can arise when there is not a suitable reagent to generate adequate contrast, for example when the latent print is on a multicoloured or dark background. More sophisticated chemical approaches developed to address this problem include spectroscopic imaging of sweat components (Worley CG, Wiltshire SS, Miller TC, Havrilla GJ, Majidi V. Detection of Visible and Latent Fingerprints Using Micro-X-ray Fluorescence Elemental Imaging. J Forensic Sci, 2006;51:57-63), reaction with secreted drug metabolites of antibody-functionalised nanoparticles (Leggett R, Lee-Smith EE, Jickells SM, Russell DA. "Intelligent" Fingerprinting: Simultaneous Identification of Drug Metabolites and Individuals by Using Antibody-Functionalized Nanoparticles. Angew Chem Int Ed, 2007;46:4100-4103) and deposition of the strongly coloured polymer (SN)ₓ (Kelly PF, King RSP, Moutimer RJ. Fingerprint and inkjet-trace imaging using disulfur dinitride. Chem Comm, 2008;46:6111-6113).

For metallic objects, of which fired cartridge cases are a forensically important example, metal vapour deposition (Migron Y, Hocherman G, Springer E, Almog J, Mandler D. Visualisation of Sebaceous Fingerprints on Fired Cartridge Cases: A Laboratory Study. J Forensic Sci, 1998;43:543-548) and the visualisation by a scanning Kelvin probe (Williams G, McMurray N. Latent fingermark visualisation using a scanning Kelvin probe. Forensic Science International, 2007;167:102-109) have proved useful. The electrodeposition of polypyrrole onto the clean metal surface between fingerprint ridges has been disclosed in Bersellini C, Garofano L, Giannetto M, Lusardi F, Mori G. Development of latent fingerprints on metallic surfaces using electropolymerisation processes. J Forensic Sci, 2001;46:871-877. WO 2008/044494 (US 2010/0047433) discloses fingerprint detection using a polymer.

All the above methods share the common characteristic of generating an image (whether a positive one of the ridge detail itself or a negative one of the spaces between the ridges) that is visually constant. The accomplishment of visual contrast is reliant upon the reagent or deposit contrasting sufficiently strongly with the substrate, a situation that is generally not guaranteed. There remains a need for improved methods for visualising a print on a surface.

According to the present invention, there is provided a method of visualising a print on a surface as defined in claim 1.

The print may be a fingerprint, ear print, lip print, palm print, foot print or shoe print. Preferably, the print is a fingerprint. It will be appreciated that the surface may carry the print.

The monomer may be an aromatic compound. One example of a suitable aromatic compound may be aniline, or a derivative thereof. The monomer may be a heterocyclic aromatic compound. Examples of suitable heterocyclic aromatic compound may be pyrrole or thiophene, or a derivative thereof. A suitable thiophene derivative may be 3,4-ethylenedioxythiophene (EDOT). Conveniently, the monomer is pyrrole, thiophene, aniline, or a derivative thereof, preferably aniline. Preferably, the monomer is aniline or a derivative thereof, preferably aniline.

Since deposition of the polymer on the surface requires application of a voltage, the surface may comprise an electrically conducting material. Hence, the surface may be a conductor or a semi-conductor substrate.

Advantageously, the surface comprises a metal. Preferably the metal is iron, steel, brass, bronze, zinc, gold, silver, platinum, tin, copper, lead, nickel, chromium, titanium, and/or aluminium, or an alloy thereof.

Conveniently, the surface forms at least part of a weapon, building, vehicle, furniture, tool, jewellery, electrical device or electronic device. Advantageously, the surface forms part of a hammer, screwdriver, crowbar, gun, cartridge, case, bullet, shell, key, lock, window, door, knife, sword, bayonet, machete, handle, seat or table.

Conveniently, the concentration of the monomer in the monomer-containing fluid is from about 0.001 M to about 1 M. The concentration of the monomer may be about 0.01 M to about 0.2 M, or from about 0.001 M to about 0.1 M. The concentration of the monomer may be about 0.01 M.

The monomer-containing fluid may have a pH between about 0 and 10, or between about 0.1 and 7, or between about 0.5 and 5. The fluid may have a substantially neutral pH. For example, in embodiments where the polymer is polypyrrole, a neutral pH is preferred, i.e. about 7. However, the monomer-containing fluid may be acidic, preferably having a pH of less than about 3. The pH of the monomer-containing fluid may be less than about 2, or less than about 1.

The monomer-containing fluid may contain an acid selected from hydrochloric acid, sulphuric acid, perchloric acid and nitric acid, preferably sulphuric acid.

Conveniently, the concentration of the acid in the monomer-containing fluid is from about 0.001 M to about 2 M. The concentration of the acid may be about 0.01 M to 1 M, or about 0.1 M to 0.5 M.

Advantageously, an electrical potential of about 0.1 V to 2 V may be applied to deposit the polymer on the surface. It will be appreciated that the value of electrical potential depends on the monomer and on the choice of reference electrode. Thus, the range of voltage provided above is determined with respect to a silver (Ag)/silver chloride (AgCl)/saturated potassium chloride (KCl) reference electrode, which should cover most monomers. The electrical potential may be about 0.5 V to 1.5 V, or about 0.7 V to 1.2 V. An electrical potential of about 0.9 V may be applied to deposit the polymer on the surface.

In one embodiment, the polymer may be deposited onto the surface using a constant potential known as "potentiostatic" deposition. In this embodiment, the voltage is kept constant when depositing the polymer on the surface.

In another embodiment, the polymer may be deposited onto the surface using a variable potential known as "potentiodynamic" deposition, in which the electric potential applied to the surface is varied over time. In this embodiment, one may scan (i.e. sweep) the potential in a linear cycle between pre-set limits, which may be about 0.1 V to 2 V to deposit the polymer on the surface, preferably -0.2 V to 1.2 V. Suitable scan rates of the potential may be about 1-1000 mVs⁻¹, or about 10-100 mVs⁻¹.

The polymer may be deposited on the surface over a period of from about 10 s to about 3600 s, preferably from about 50 s to about 3000 s, or from about 200 s to about 2400 s. The polymer may be deposited over a period of about 200 s to about 400 s.

Conveniently, the monomer-containing fluid is removed from the surface before the step of applying an electrical potential to the polymer on the surface to visualised the print.

Advantageously, a fluid that is substantially free of the monomer is contacted with the surface before the step of applying an electrical potential to the polymer on the surface to visualised the print.

The fluid that is substantially free of the monomer may have a pH between about 0 and 10, or between about 0.1 and 7, or between about 0.5 and 5. The fluid may have a substantially neutral pH, i.e. about 7. For example, in embodiments where the polymer is polypyrrole, a neutral pH is preferred. However, the fluid that is substantially free of the monomer may be acidic, preferably having a pH of less than about 3. The pH may be less than about 2, or less than about 1.

Conveniently, the fluid that is substantially free of the monomer contains an acid selected from hydrochloric acid, sulphuric acid, perchloric acid and nitric acid, preferably sulphuric acid.

Advantageously, the concentration of the acid in the fluid that is substantially free of the monomer is from about 0.001 M to about 2 M. The concentration of the acid may be about 0.01 M to 1 M, or about 0.1 M to 0.5 M.

The step of applying an electrical potential to the surface may result in a change in colour of the polymer film. This colour change may be referred to as an electrochromic effect.

Conveniently, the colour of the polymer film is yellow, green or blue. It will be appreciated that such colours represent the case for example when using polyaniline. However, it is also envisaged that other colours (e.g. red) may be generated using other polymers defined herein. The skilled person will appreciate that other colours could be generated through judicious choice of monomers and substituent effects.

Advantageously, an electrical potential of from about -1 V to about 2 V is applied to the surface to visualise the print. The electrical potential may be about -0.5 V to about 1.5 V. More preferably, the electrical potential may be about -0.2 V to about 0.9 V.

The electrical potential applied to the surface to visualise the print may be varied over time.

Advantageously, the surface forms the working electrode in a three-electrode cell.

The thickness of the polymer on the surface may be from about 20 nm to about 3µm, preferably from about 100 nm to about 2.5 µm, more preferably from about 140 nm to about 2 µm, and most preferably from about 300 nm to about 800 nm.

Conveniently, the method further comprises the step of recording an image of the visualised print.

Advantageously, the method further comprises the step of analysing the visualised print.

The method may further comprise the step of comparing the visualise print with another print.

In some embodiments, for example where the surface is non-conducting (e.g. plastic or paper or some other absorbent material), it may not be possible to effectively visualise the print upon application of the voltage. Thus, in such embodiments, the method may comprise transferring the print from a non-conducting surface to a conducting surface, on which it is possible to visualise the print upon application of the electrical potential. The method may comprise the use of a suitable transferring means for transferring the print to a conducting material. The transferring means may comprise adhesive, which is capable of picking up the print from the non-conducting surface and then applying the print onto the conducting surface. For example, the transferring means may comprise sticky tape, or the like.

Conveniently, the method may further comprise removing the polymer from the surface.

According to another aspect of the present invention, there is provided a surface comprising a polymer obtainable by the method of the invention.

According to a further aspect of the invention, there is provided a polymer obtainable by the method of the invention.

According to a yet further aspect of the invention, there is provided a visualised print obtainable by the method of the invention.

According to another aspect of the invention, there is provided a kit for visualising a print comprising a monomer-containing fluid and directions for performing the method of the invention.

According to yet another aspect of the invention, there is provided an image of a visualised print resulting from the method of the invention.

All of the features described herein (including any accompanying claims, abstract and drawings), and/or all of the steps of any method or process so disclosed, may be combined with any of the above aspects in any combination, except combinations where at least some of such features and/or steps are mutually exclusive.

The invention will now be described by way of example with reference to the accompanying figures, in which:
Figure 1a is a photograph of an electrochromically enhanced sebaceous fingerprint on stainless steel viewed with sample maintained under potential control at -0.2 V,
Figure 1b is a photograph of an electrochromically enhanced sebaceous fingerprint on stainless steel viewed with sample maintained under potential control at 0.4 V,
Figure 1c is a photograph of an electrochromically enhanced sebaceous fingerprint on stainless steel viewed with sample maintained under potential control at 0.6 V,
Figure 1d is a cyclic voltammogram of polyaniline film deposited on fingerprinted stainless steel with a scan rate of v = 20 mV s⁻¹,
Figure 2 is a chart showing the distribution of print sample quality before and after electrochromic enhancement,
Figure 3a is an optical microscope image of an electrochromically enhanced fingerprint showing a core,
Figure 3b is an optical microscope image of an electrochromically enhanced fingerprint showing a ridge ending,
Figure 3c is an optical microscope image of an electrochromically enhanced fingerprint showing a lake,
Figure 3d is an optical microscope image of an electrochromically enhanced fingerprint showing a bifurcation,
Figure 4 is a schematic illustration of an electrochemical cell for use in the present invention,
Figure 5a is a photograph of a sebaceous fingerprint on lead,
Figure 5b is a photograph of a polyaniline electrochromically enhanced sebaceous fingerprint on lead viewed *ex situ* after emersion at 0.8 V,
Figure 6a is a photograph of a polyaniline electrochromically enhanced sebaceous fingerprint on stainless steel viewed *ex situ* after emersion at 0.7 V,
Figure 6b is a photograph of a polyaniline electrochromically enhanced sebaceous fingerprint on platinum viewed *ex situ* after emersion at 0.5 V,
Figure 7a is a schematic illustration of the chemical structure of poly-3,4-ethylenedioxythiophene (PEDOT) derived from its monomer 3,4-ethylenedioxythiophene,
Figure 7b is a photograph of a PEDOT electrochromically enhanced sebaceous fingerprint on stainless steel viewed *ex situ* after emersion at 0.8 V,
Figure 8a is a schematic illustration of the chemical structure of poly-3,4-ethylenedioxythiophene (PEDOT) (top), and poly-aniline (PAn) (bottom),
Figure 8b is a chart showing the percentage of forensically usable prints (i.e. grade 3 or 4 according to Bandey scale) after various treatments of the fingerprint, including dusting, superglue, wet powder, electrochromic enhancement with EDOT, electrochromic enhancement with poly-aniline (PANI) and no treatment (control),
Figure 8c is a chart showing the same data as Figure 8b, except the data for each treatment is normalized by dividing them by the no treatment (control),
Figure 9a is a bar chart showing the percentage of usable quality prints on 1 day old samples when the prints have been heated in an oven at 150°C for the entire period of time,
Figure 9b is a bar chart showing the quality of usable prints on 1 dray old samples previously washed with soapy water and then stored at ambient conditions (in air, at room temperature) for the remaining period of time,
Figure 9c is a bar chart showing the quality of prints on 7 day old samples when the prints have been heated in an oven at 150°C for the entire period of time, and
Figure 9d is a bar chart showing the quality of prints on 7 day old samples previously washed with soapy water and then stored at ambient conditions (in air, at room temperature) for the remaining period of time.

The present invention relates to a novel strategy for enhancement of latent prints, including fingerprints, based on the concept of reversibly controlling the visual contrast between the deposited ridge detail and the deposit-free substrate between the ridges. In particular, the present invention is directed towards the electrochromic enhancement of latent fingerprints on metal surfaces.

In this specification, the term "print" may mean a distinctive mark or pattern left on a surface through contact with another entity, preferably an animate entity such as a human. The print may result from contact of the surface with part of a human body, or from contact with an item carried by or on a human, such as a print left by footwear.

One goal addressed by the invention is the spatially selective deposition on the intervening bare substrate of a print of a material whose optical properties can be reversibly manipulated so as to generate a range of colours, at least one of which will necessarily contrast with the undecorated ridge detail.

The strategy is demonstrated for the case of a fingerprint on a metal surface upon which an electrochromic polymer is electrochemically deposited. Stainless steel was chosen as the substrate in view of its widespread use in common implements (handles, tools, knives) relevant to forensic evidence. Visualisation of electrochromically enhanced fingerprints according to the method of the invention were also demonstrated on lead and platinum.

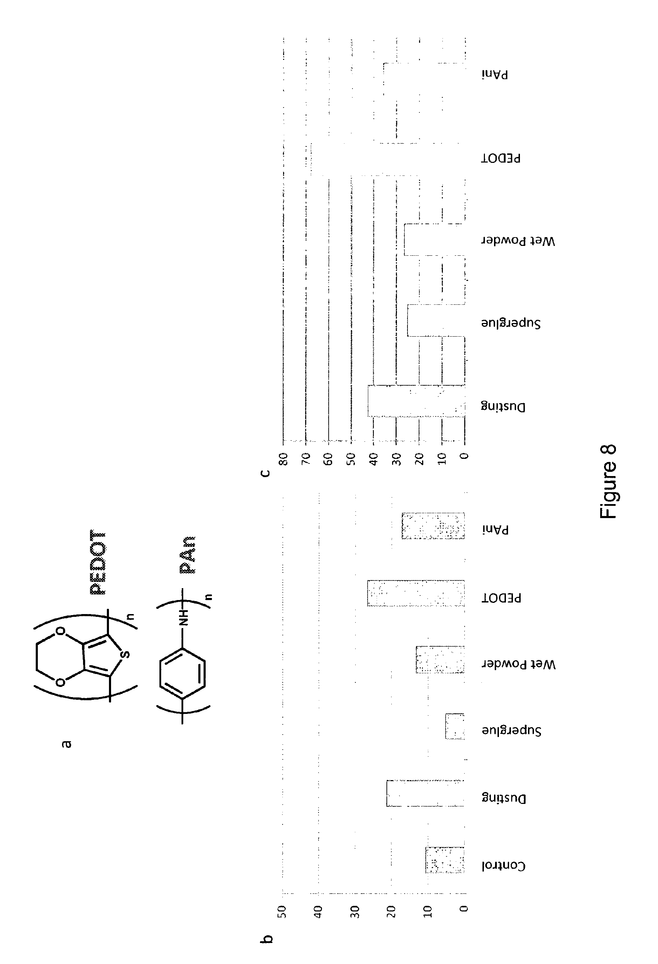

The monomer may be aniline and the chemical structure of polyaniline is shown in Figure 8a. The choice of polyaniline as the electrochromic polymer was based on the facility to operate in aqueous media, its exceptional chemical stability and general robustness in thin film form, and the opportunity to access three optically distinct oxidation states (Handbook of Conducting Polymers. Skotheim T.A., Elsenbaumer R.L., Reynolds J.R. Eds.; Parcel Dekker: New York, 1998, and Multicoloured electrochromism polymers: structures and devices. Argun AA, Aubert PH, Thompson BC, Schwendeman I, Gaupp CL, Hwang J, Pinto NJ, Tanner DB, MacDiarmid AG, Reynolds JR. Chem. Mater, 2004:16:4401-4412). Other electrochromic polymers could be used, including polypyrrole and polythiophene and their derivatives.

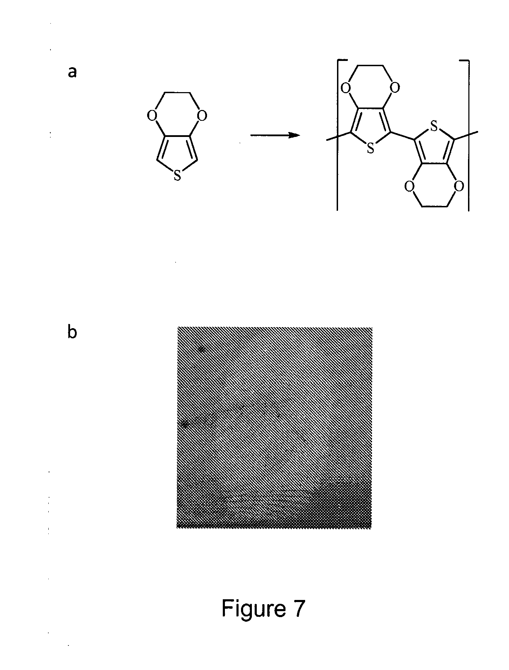

The monomer may also be a derivative of thiophene, such as 3,4-ethylenedioxythiophene (PEDOT). Poly-3,4-ethylenedioxythiophene (PEDOT), like polyaniline, is an electrochromic, conducting polymer which can be oxidised and reduced electrochemically. The chemical structure of PEDOT is shown in Figure 7a and 8a.

The approach exploits the fact that the fingerprint deposit locally "masks" the surface; an insulating deposit as little as a few nanometres thick is sufficient to block the electron transfer upon which polyaniline deposition is reliant and the typical fingerprint deposit of as little as 10 nm to as much as 2 µm (according to load) is more than adequate to accomplish this. Polyaniline has been widely explored for application in electrochromic devices (see Argun AA *et al.*) in which the goal is the exact opposite of that here, namely laterally *uniform* colouration of the surface, be it an element of a display device or an architectural glazing unit.

As a consequence of this body of work, the electrochemical and optical properties of polyaniline are well known: the material has three oxidation states that can be accessed at conveniently spaced intervals of applied voltage within the potential window accessible in aqueous acidic media. Additionally, the participation of both proton (cation) and anion transfers (Hillman AR, Mohamoud AM. Ion, solvent and polymer dynamics in polyaniline conducting polymer films. Electrochim. Acta, 2006:51:6018-6024) to maintain electroneutrality upon changing polymer charge state generally ensures rapid switching of charge and optical states in aqueous media.

The enhancement of latent fingerprints on a metallic (e.g. stainless steel, lead or platinum) surface is described. The approach relies on the inhibition of electrochemical processes at regions of the surface masked by the fingerprint deposit. Electropolymerisation of aniline or EDOT on non-masked regions of the surface is used to generate an electroactive polymer film that is a negative image of the fingerprint. Upon transfer of the polymer-coated surface to monomer-free aqueous electrolyte, the electrochromic properties of the polyaniline or PEDOT film can be realized.

By variation of the applied potential, the polymer colour can be adjusted to optimise the visual contrast between the fingerprint ridge deposit and the inter-ridge polymer deposit. At the simplest level this may involve selection from the three available colours, i.e. oxidation states. Where an excess of polymer is deposited this may be remedied via decreased optical density, i.e. extent of film oxidation; this may be useful in cases where replicate samples are not available.

Assessment of a selection of latent fingerprints, representing the full range of quality in a recognized grading scheme, showed that this electrochromic enhancement procedure transformed a significant fraction of forensically unusable images into usable images.

The method of the invention involves the deposition of a polymer onto an electrically-conducting surface on which a print is present. This involves contacting the surface with a monomer whilst applying an electrical potential. This causes the selective deposition of polymer onto the areas of the surfaces that are not covered by the print. The selective deposition of the polymer in these areas is believed to be influenced by the electrically insulating properties of the residue forming the print.

Deposition of the polymer is continued until enough polymer has been deposited to enhance the print on the surface. If the deposition process is continued beyond this point, then the thickness of the polymer layer may become equal to or greater than the thickness of the residue that forms the ridges of the print. This may result in reduced resolution and/or visualisation of the print.

The general methodology of the present invention will now be described by way of example.

### Examples

### Example 1

The first stage is the deposition of polymer, as follows:
- Three electrodes; an optional saturated potassium chloride (KCl) reference electrode, a platinum counter electrode and a working electrode (e.g. steel sample to deposit on to). Other electrochemical arrangements could be used, such as a two electrode cell without using the reference electrode. The use of a reference electrode allows for more precise control of the electrochemical conditions.
- Solution made; e.g. 25 ml of 1 M H₂SO₄, 0.1 M aniline and added to cell.
- Constant potential 0.9 V applied between about 200 and about 2700 seconds, typically 400 seconds, until ridge detail is apparent or becomes apparent that no detail is possible.
- Working electrode removed from cell and rinsed in deionised water.

The next stage is variation of the colour of the polymer to enhance print details:
- Three electrode cell; a reference electrode which may be silver (Ag)/silver chloride (AgCl)/saturated KCl, a platinum counter electrode and a working electrode (e.g. steel sample from the first stage).
- Monomer free solution made; e.g. 25 ml of 1 M H₂SO₄.
- Cycle the sample (-0.2) V to (0.9) V, usually 10 scans at 0.02 V s⁻¹.

In order to optimise the method of the invention, the following observations may be made:
- The typical thickness of the polymer deposit can vary between about 70 nm and about 2400 nm, with a typical thickness of about between about 500 nm and about 600 nm for fingerprints. The polymer fills between the ridges and then spills onto the ridges, so it is normally optimal to stop the process when the print is properly visualised.
- A camera image taken of the electrochromically enhanced print is normally a good representation of the colour change.
- When running at constant potential (e.g. -0.2, 0.3, 0.9 V etc), the electrochromic change in colour of a suitable polymer layer normally takes less than about 10 seconds. In other words, this is how long it takes for the polymer to adjust to its new colour when the potential is changed.

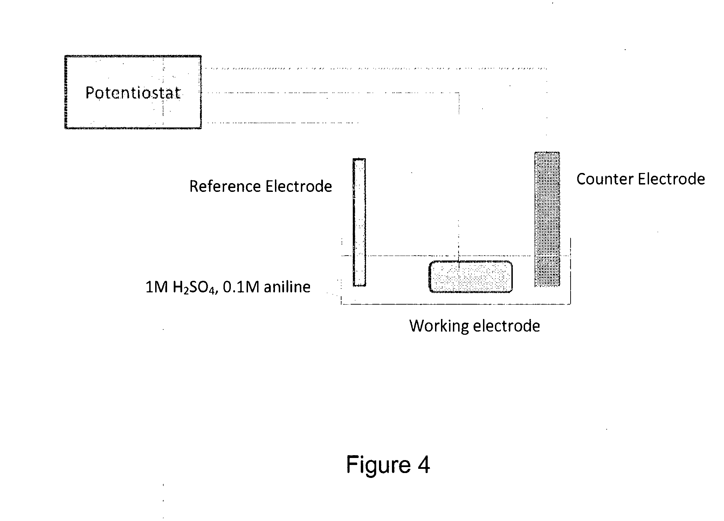

A typical apparatus for performing the present invention is shown in Figure 4. The sample having a print on its surface forms the working electrode (e.g. steel) of a three-electrode electrochemical cell. The sample is placed in contact with a monomer-containing fluid, such as an aqueous solution of 0.1 M aniline in 1 M sulphuric acid. The electrochemical cell also comprises a platinum counter electrode and a Ag/AgCl/saturated KCl reference electrode. All of the three electrodes are connected to a potentiostat. As mentioned above, other arrangements could be used, such as the use of a two-electrode cell without a reference electrode, as known in the art.

The polymer may be deposited on the surface by potentiodynamic means, i.e. by using potential as the controlled parameter but linearly cycled between pre-set limits using a potentiostat, which may be between -0.2V to 1.2V. Suitable scan rates of the potential may be about 1-1000 mVs⁻¹.

Alternatively, the polymer may be deposited on the surface under conditions of controlled current, rather than controlled potential. In other words, polymer may be deposited when the current can be controlled directly (a galvanostatic experiment, run using an instrument called a galvanostat) or indirectly by controlling the potential and letting the current assume whatever value corresponds to that potential (a potentiostatic experiment, run using a potentiostat).

### Example 2

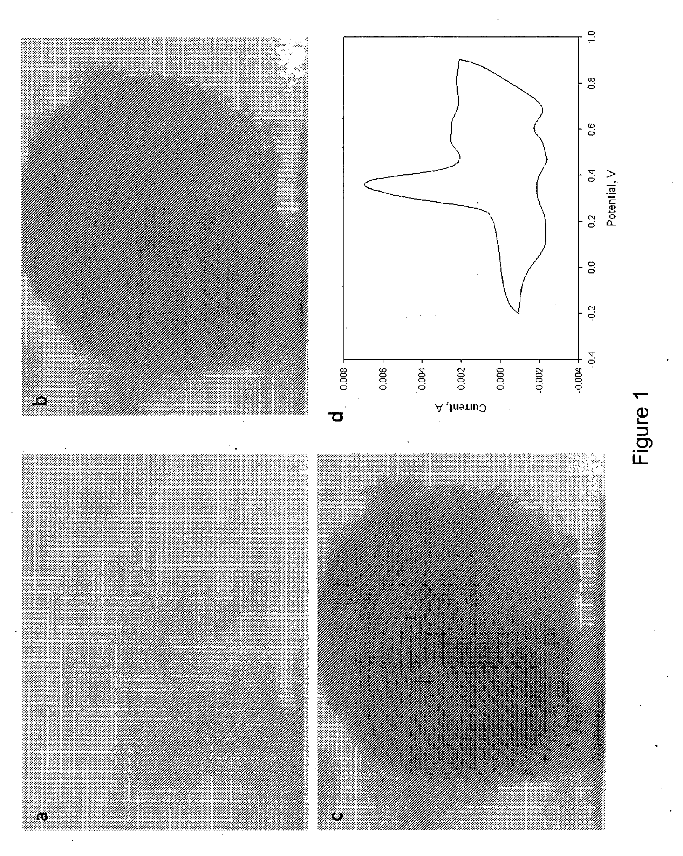

The outcome of a representative fingerprint enhancement on a stainless steel substrate is shown in Figures 1a, 1b and 1c. The initial print, derived from sebaceous sweat, was visible with the aid of suitable lighting and viewing angle but details could not be discerned and identification would not have been possible based on a photographic image. Images of the polymer-enhanced fingerprint were acquired at 100 mV intervals during a redox cycle. These figures show selected images at applied potentials generating distinct colours of the polyaniline film.

As will be described below in the examples, polymer was deposited on stainless steel samples (polymer film deposition time 450 s), with the sample subsequently being immersed in 1 M H₂SO₄ and maintained under potential control to enhance and vary the colour of the polymer deposit. Figure 1a, 1b and 1c are photographs of the same sebaceous fingerprint on stainless steel that has been electrochromically enhanced and visualised under different applied electrical potentials.

Figure 1 a shows the fingerprint maintained under potential control at -0.2 V, with the polymer deposit having a yellow colour. Figure 1b shows the fingerprint maintained under potential control at 0.4 V, with the polymer deposit having a green colour. Figure 1c shows the fingerprint maintained under potential control at 0.6 V, with the polymer deposit having a blue colour.

These figures demonstrate the ability to vary the optical properties of the polymer deposit on the sample, which allows for optimisation of the contrast between the polymer and the surface. This technique allows latent prints to be visualised. The technique also allows the improvement in quality of prints on a wide range of surfaces.

Figure 1d shows a typical cyclic voltammogram of polyaniline film deposited on fingerprinted stainless steel; scan rate, v = 20 mVs⁻¹. This figure shows a representative voltammogram of the polymer films deposited: the response for this laterally inhomogeneous film is consistent with those widely reported for laterally homogeneous films deposited on bare electrodes (see Argunn AA *et al.* and Hillman AR *et al.*). Although the fingerprint deposit clearly locally inhibits the electrochemistry that underlies polymer deposition and redox switching, it does not extend to the intervening inter-ridge areas to prevent polymer deposition and redox switching.

At the cathodic extreme of the potential range, at which point the polymer has the palest colour and thus contrasts least with the fingerprint deposit, the polymer-enhanced print barely shows recognizable features. As the potential is made more positive, the polymer darkens and the contrast with the fingerprint becomes more marked. At the anodic extreme of the potential range, there is sharp visual contrast between the polyaniline "infill" and the initial fingerprint deposit.

For substantially thicker films, the clarity of the image was less good and the majority of the surface had the colour of polyaniline. This is believed to result from the over-filling of the trenches between fingerprint ridges, to the extent that the polymer grows across the surface of the fingerprint ridge.

Based on the integrated current and an estimate of the fraction of the surface available for polymer deposition, it is estimated that the polymer film thickness for the samples studied were in the range of from about 140 nm to about 2 µm, according to the individual deposition times used. The height of a fingerprint deposit can vary widely, such as from 10 nm to 2 µm (Thomas GL, Reynoldson TE. Some observations on fingerprint deposits, J. Phys. D: Appl. Phys, 1975;8:724-729), so the present experiments span a range from low trench filling to substantial trench over-filling.

### Experimental Procedures

The substrates used in this example were stainless steel plates (area 6.25cm², thickness 0.8mm), which were polished to generate a mirror-like surface finish (with Brasso™ metal polish for approximately 5 min), washed in warm soapy water and left to dry in air. Prior to fingerprint deposition, the subject's hands were thoroughly washed with soap, rinsed with water and dried. To produce a sebaceous print the fingertips were rubbed around the nose and forehead region; an even spread of perspiration was achieved by rubbing the fingertips together and over the palm of the hand. To produce an eccrine print, a powder-free nitrile glove was worn for 15 minutes. In both cases, fingerprints were deposited on the substrates by application of minimal pressure.

Polyaniline films were deposited potentiostatically (0.9 V) on the stainless steel substrates as the working electrode in a conventional three electrode cell, with a saturated KCl calomel reference electrode and a platinum counter electrode. The deposition solution contained 0.1 M aniline in aqueous 1 M H₂SO₄. Deposition times were in the range of from 200 to 2400 seconds. Subsequent fingerprint visualisation was carried out potentiodynamically (-0.2 < E/V < 0.9; scan rate, v = 0.02 V s⁻¹) in monomer-free aqueous 1 M H₂SO₄. The fingerprint images were acquired using a Pentax Optio M40 digital camera and a Meiji Techno MT7100 trinocular microscope.

### Example 3

The goal of the study was fingerprint enhancement, to the extent that forensically unusable prints might be made usable. In order to assess the efficacy of the polymer enhancement, the quality of the prints as deposited and subsequent to polymer-enhancement were graded according to a scale devised by Bandy (Bandey HL. Fingerprint development and imaging newsletter: the powders process, study 1. Sandridge: Police Scientific Development Branch, Home Office, 2004; Report No.:54/04). The grading system for quality of fingerprint ridge detail disclosed by Bandey is summarised in Table 1 below:

**Table 1 Bandey grading scheme for quality of fingerprint ridge**

| **Grade** | **Fingermark Characteristic** |
|---|---|
| 0 | No development |
| 1 | No continuous ridges; all discontinuous or dotty |
| 2 | One third of mark comprises continuous ridges; remainder shows no development or is dotty |
| 3 | Two thirds of mark comprises continuous ridges; remainder shows no development or is dotty |
| 4 | Full development: whole mark comprises continuous ridge detail |

A set of 50 latent fingerprint samples was prepared, using small variations in the protocol in Example 2 to generate variations in initial grading from 0 to 4. These variations, generated by different sweat inducement times, individual donors, ambient conditions and deposition pressure, and aged for different times (2 hours to 39 days), represent a plausible spectrum of print quality. The same prints were graded after polyaniline enhancement. Since the darkest polymer state is accessed at 0.9 V, samples were assessed *in situ* commencing at this applied potential and then at 0.1 V decrements down to -0.2 V. This has the practically important attribute of exploiting maximum optical density (accessed at higher potential) for under-filled trenches and lesser optical density (accessed at lower potential) for fully- or over-filled trenches.

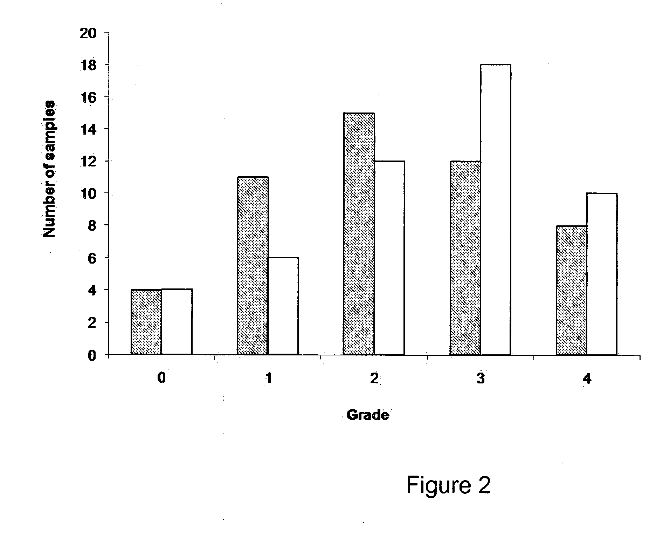

The outcomes of electrochromic enhancement of thee latent fingerprint samples are summarized in Figure 2, which is a chart showing the distribution of sample quality according to the scheme of Bandey, discussed above. Shaded bars represent fingerprint samples as deposited (not enhanced) and viewed *ex situ.* Open bars represent the same samples after electrochromic enhancement and viewed *in situ* under potential control. The critical feature is a shift of the mode from grade 2 (not useful) to grade 3 (useful). A numerical assessment shows that 20 of the initial prints were grade 3 or 4, but enhancement raised this to 28, i.e. an increase in the number of useable fingerprints by 40%. This is clearly a significant improvement in the overall quality of the sample of prints.

### Example 4

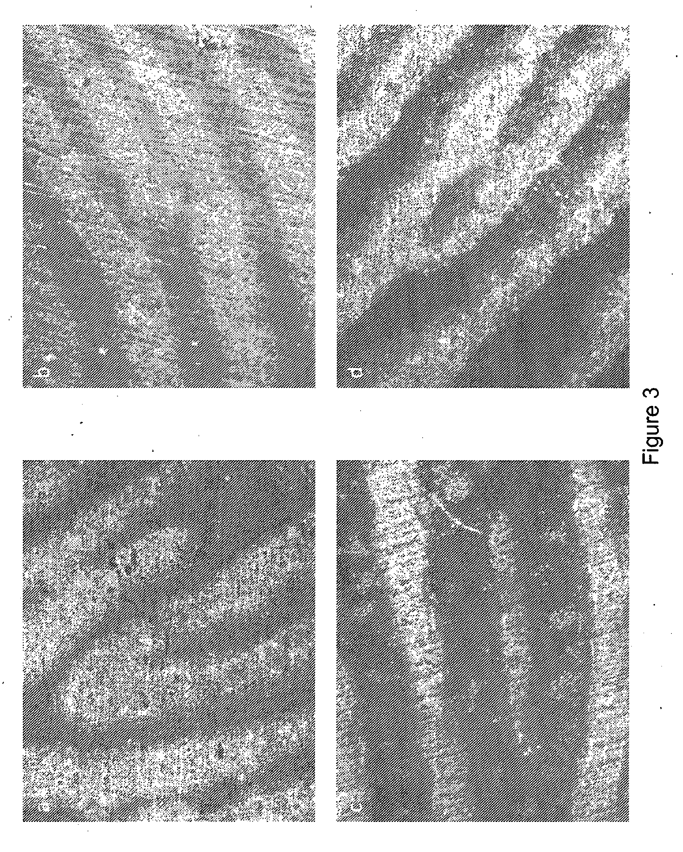

In the present invention, polymer is electrodeposited solely in the trenches between ridge deposits, with the objective of visualizing two spatially segregated regions - the ridge detail and intervening spaces. Examples of electrochromically enhanced prints viewed under an optical microscope reveal clear secondary level detail, as illustrated by the core, ridge ending, lake and bifurcation features shown in Figures 3a, 3b, 3c and 3d respectively.

The optical microscope images in Figures 3a to 3d of sebaceous fingerprint on stainless steel, viewed *ex situ*: Figure 3a core (polymer deposition time 400 s, sample emersion at 0.74V), Figure 3b ridge ending (polymer deposition time 400 s, sample emersion 0.74 V), Figure 3c lake (polymer deposition time 400 s, sample emersion at 0.82 V), Figure 3d bifurcation (polymer deposition time 400 s, sample emersion at 0.67 V).

Figure 3a shows a magnified view of a core feature of a fingerprint on a stainless steel sample. The electropolymerisation of the aniline monomers from the acidic solution has occurred on the parts of the steel surface free from the sebaceous residue left by the fingerprint, creating a negative image of the ridges of the print. The application of an electrical potential to the polyaniline deposit has hallowed optimisation of the visual contrast between the colour of the deposit and the underlying surface area that is free from the polymer. Figure 3a shows the clear visual difference between the polymer deposit and the print, allowing the identification of features of the print.

Figure 3b clearly shows details of the fingerprint, including a ridge ending. Figure 3c shows that characterising features of fingerprints such as a lake are identifiable from latent prints using the present invention. Similiarly, Figure 3d shows the bifurcation of a ridge in the electrochromatically enhanced negative polymer image of the print.

Furthermore, closer inspection of the images resulting from the method of the present invention can reveal aspects of tertiary detail, such as the images of pores from which the sweat is secreted. Although not widely used in practice at this time, there is interest in accessing this additional level of detail for identification purposes, particularly in the case of partial fingerprints, for which the volume of information is necessarily smaller. The fact that this third level detail is more readily visible after the polymer enhancement process of the present invention indicates that the interaction of the monomer and/or polymer with the sample is more sophisticated than simple exclusion from the ridges and deposition on the intervening spaces.

### Example 5

The present invention allows visualisation of latent fingerprints on various metal surfaces, such as stainless steel, lead and platinum. Fingerprint samples were prepared following the potentiostatic protocol in Example 2 for stainless steel, and analogous potentiodynamic protocols (potential limits -0.2 V and 1.2 V; scan rates 10-100 mV s⁻¹) for lead plates and platinum.

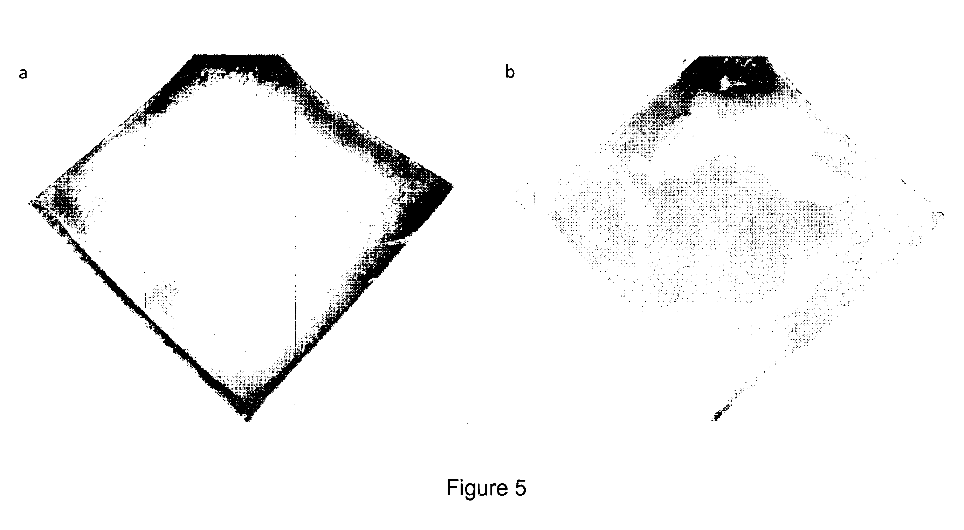

The outcome of a representative latent fingerprint on a lead substrate is shown in Figure 5a and the same fingerprint following electrochromic enhancement using the method of the invention is shown in Figure 5b. Referring to Figure 5a, the initial print, derived from sebaceous sweat, was not visible on the lead substrate and thus identification would not have been possible based on the photographic image. Following electrochromic enhancement, however, the fingerprint became visible on the lead substrate allowing identification (Figure 5b).

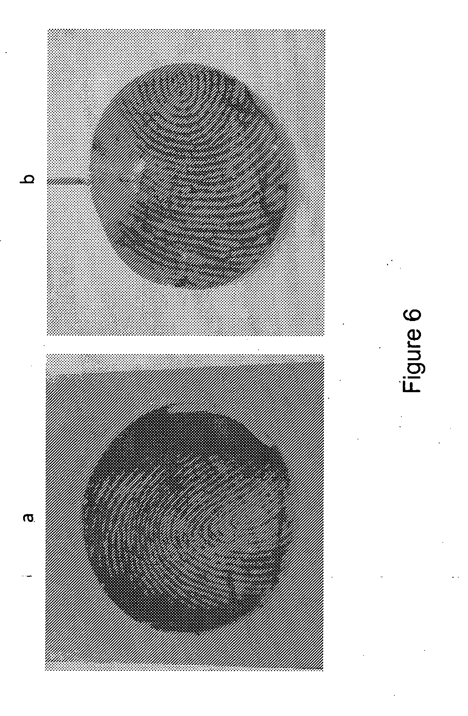

The outcome of a representative latent fingerprint on a stainless steel substrate following electrochromic enhancement using the method of the invention is shown in Figure 6a. A latent fingerprint on platinum substrate following electrochromic enhancement is shown in Figure 6b.

### Example 6

In the present invention, a preferred electrochromic polymer may be poly-3,4-ethylenedioxythiophene (PEDOT). Figure 7b shows a representative photograph of a sebaceous fingerprint on a stainless sample which has been enhanced electrochromically using PEDOT.

The electrodeposition of PEDOT was achieved in a solution containing EDOT (0.01 M), sodium dodecylsulphate/SDS (0.01 M) and H₂SO₄ (0.1 M), at a potential of 0.9 V. Deposition times ranged from 140 to 3600 s. Potentiodynamic methods used for fingerprint visualisation were performed in monomer free H₂SO₄ (0.1 M). Potential limits range from -0.8 V to 0.8 V at a scan rate of 30 mV s⁻¹.

### Example 7

The quality of fingerprint enhancement provided by the present invention was compared with enhancement using common methods such as dusting with a powder, cyanoacrylate fuming ("superglue") and wet powder.

In this experiment, a set of 600 fingerprint samples was prepared following the protocol set out in Example 2. The fingerprints were prepared from 5 different donors (3 males and 2 females), and the fingerprints from each donor were stored and treated in exactly the same manner.

The fingerprint samples were exposed to the following environments, which have been designed to mimic realistic situations:
1. Ambient (air, room temperature);
2. Oven at 150 °C for the entire period;
3. Stored under water for the entire period;
4. Acetone wash followed by ambient storage for the remainder of the period; and
5. Soap wash followed by ambient storage for the remainder of the period.

The fingerprint samples were exposed in each of the above environments until enhancement on day 1, day 7 and day 14 after the deposition of the fingerprint. The fingerprints were enhanced using the following methods:
1. Control (no treatment);
2. Dusting with a powder;
3. Cyanoacrylate fuming ("superglue");
4. Wet powder;
5. Electrochromic enhancement using PEDOT; and
6. Electrochromic enhancement using polyaniline (PAni).

The quality of each of the enhanced fingerprints was assessed according to the Bandey grading scheme (Table 1).

The results of this experiment are presented in Figure 8. Figure 8b shows a summary of the data represented in a bar chat. The chart shows the percentage of enhanced prints that is forensically usable (i.e. Bandey scale grades 3 and 4) for each enhancement method. The data for each enhancement method and the control were averaged over the 5 environments to which the samples were exposed.

The inventors note that the absolute percentages are not the key feature, since the prints were taken with a range of initial grades (as assessed on the Bandey scale), but instead, it is the relative percentages (i.e. enhancements) that are important. Therefore, the data from this experiment were normalised by dividing the percentage of enhanced prints that is forensically usable for each enhancement method by the control. Figure 8c shows the normalised performance of the various enhancement methods.

Referring to Figures 8b and 8c, it can be seen that PEDOT polymer enhancement performs best, with the highest percentage of forensically usable prints. Polyaniline polymer enhancement (overall) is comparable to simple dusting.

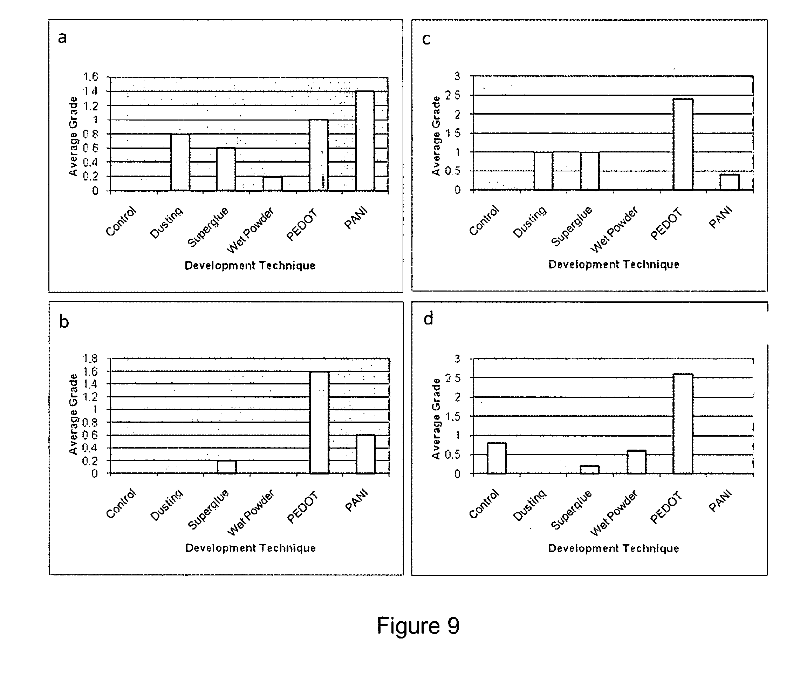

However, these averages conceal the fact that the polymer enhancement performed well when the fingerprints have been exposed to specific environments. For example, Figure 9a and 9c show the percentage of enhanced prints that were forensically usable when the prints have been exposed to heating in a low temperature oven. Figures 9b and 9d show the results for the percentage of enhanced prints that were forensically usable when the prints have been washed with soapy water.

Both electrochromic polymers, PEDOT and polyaniline, generally perform well with prints that have undergone heating and washing with soap. It is also clear that they perform differently for samples with different histories, for example, polyaniline is better than PEDOT for 1 day old samples exposed to heat, but PEDOT is better for older (7 day) samples.

PEDOT appears particularly effective for samples that have been washed with soapy water. It appears that washing the fingerprint sample with soapy water has removed a large amount of the fingerprint residue. The small amount of fingerprint residue left on the sample does not appear to be sufficient for most of the enhancement methods (i.e. dusting, superglue and wet powder) to produce forensically usable prints. However, there seems to be enough of the print residue present on the sample to act as a "mask" for PEDOT deposition and thereby achieve an enhanced print that is forensically usable.

Therefore, the inventors envisage the polymer enhancement as being particularly relevant to evidence samples that have been exposed to certain environments.

In the context of the numerous practical applications of this approach, it is worth noting that while acquisition of fingerprints from wet porous surfaces is routinely accomplished, relatively few techniques are applicable to wet non-porous surfaces (Zhang M, Girault H H. SECM for imaging and detection of latent fingerprints. Analyst, 2009;134:25-30 and Cuce P, Polimeni G, Lazzaro A P, De Fulvio G. Small particle reagents technique can help to point out wet latent fingerprints. Forensic Science International, 2004;146:6-7). Since the present method can be applied in the presence of an aqueous medium, previous wetting of the sample would of itself not be a problem. A limitation of cyanoacrylate ("superglue") fuming is that the quality of the developed print decreases as the fingerprint ages. While this can be remedied by an enhancement agent, such as acetic acid (Wargacki S P, Lewis L A, Dadmun M D. Enhancing the Quality of Aged Latent Fingerprints Developed by Superglue Fuming: Loss and Replenishment of Initiator. J Forensic Sci, 2008;53:1138-1144), this represents an additional step. The exemplary polymers polyaniline and PEDOT are known to have exceptional stability under ambient conditions and indeed it has been found here that a usable fingerprint image is equally acquirable from aged or fresh fingerprint deposits without additional steps or reagents.

The listing or discussion of an apparently prior-published document in this specification should not necessarily be taken as an acknowledgement that the document is part of the state of the art or is common general knowledge.

## Claims

1. A method of visualizing a print on a surface, comprising the steps of:
- providing a surface that comprises a print,
- contacting the surface with a monomer-containing fluid,
- passing an electrical current between the surface and the fluid to deposit electrochromic polymer on the surface, and
- applying an electrical potential to the surface to visualise the print.

2. A method according to claim 1, wherein the print is a fingerprint, ear print, lip print, palm print, foot print or shoe print.

3. A method according to either claim 1 or 2, wherein the monomer is an aromatic compound or, a heterocyclic aromatic compound, optionally wherein the monomer is aniline or a derivative thereof, or pyrrole or thiophene, or a derivative thereof, such as 3,4-ethylenedioxythiophene (EDOT).

4. A method according to any preceding claim, wherein the surface comprises an electrically-conducting material, optionally a metal, wherein the metal is iron, steel, brass, bronze, zinc, gold, silver, platinum, tin, copper, lead, nickel, chromium, titanium, and/or aluminium, or an alloy thereof, optionally wherein the surface forms at least part of a weapon, building, vehicle, furniture, tool, jewellery, electrical device, electronic device, hammer, screwdriver, crowbar, gun, cartridge, case, bullet, shell, key, lock, window, door, knife, sword, bayonet, machete, handle, seat or table.

5. A method according to any preceding claim, wherein the concentration of the monomer in the monomer-containing fluid is from about 0.001 M to about 1 M, wherein the monomer-containing fluid has a pH between 0 and 10, preferably having a pH of less than about 3, optionally wherein the monomer-containing fluid contains an acid selected from hydrochloric acid, sulphuric acid, perchloric acid and nitric acid, preferably sulphuric acid, optionally wherein the concentration of the acid in the monomer-containing fluid is from about 0.001 M to about 2 M.

6. A method according to any preceding claim, wherein an electrical potential of about 0.1 V to 2 V is applied to deposit the polymer on the surface, and the potential is kept constant or varied over time.

7. A method according to any preceding claim, wherein the monomer-containing fluid is removed from the surface before the step of applying an electrical potential to the polymer on the surface to visualise the print, optionally wherein a fluid that is substantially free of the monomer is contacted with the surface before the step of applying an electrical potential to the polymer on the surface to visualise the print, optionally wherein the fluid that is substantially free of the monomer has a pH of about 0 to 10, preferably less than about pH 3, optionally wherein the fluid that is substantially free of the monomer contains an acid selected from hydrochloric acid, sulphuric acid, perchloric acid and nitric acid, preferably sulphuric acid, optionally wherein the concentration of the acid in the fluid that is substantially free of the monomer is from about 0.001 M to about 2 M.

8. A. method according to any preceding claim, wherein the step of applying an electrical potential to the surface results in a change in colour of the polymer film, optionally wherein the colour of the polymer film is yellow, green, red or blue.

9. A method according to any preceding claim, wherein an electrical potential of from about -1 V to about 2 V is applied to the surface to visualise the print, optionally wherein the electrical potential applied to the surface to visualise the print is varied over time, optionally wherein the surface forms the working electrode in a three-electrode cell, optionally wherein the thickness of the polymer on the surface is from about 20 nm to about 3 µm, preferably from about 100 nm to about 2.5 µm, more preferably from about 140 nm to about 2 µm, and most preferably from about 300 nm to about 800 nm.

10. A method according to any preceding claim, further comprising the steps of:
- recording an image of the visualised print,
- analysing the visualised print,
- comparing the visualised print with another print,
- transferring the print from a non-conducting surface to a conducting surface, preferably using a suitable transferring means, and
- removing the polymer from the surface.

## Patentansprüche

1. Verfahren zum Sichtbarmachen eines Abdrucks auf einer Oberfläche, die folgenden Schritte umfassend:
- Bereitstellen einer Oberfläche, die einen Abdruck umfasst,
- In-Kontakt-Bringen der Oberfläche mit einem monomerhaltigen Fluid,
- Leiten eines elektrischen Stroms zwischen der Oberfläche und dem Fluid, um elektrochromes Polymer auf der Oberfläche abzuscheiden, und
- Anlegen eines elektrischen Potenzials an die Oberfläche, um den Abdruck sichtbar zu machen.

2. Verfahren nach Anspruch 1, wobei der Abdruck ein Fingerabdruck, ein Ohrabdruck, ein Lippenabdruck, ein Handflächenabdruck, ein Fußabdruck oder ein Schuhabdruck ist.

3. Verfahren nach Anspruch 1 oder 2, wobei das Monomer eine aromatische Verbindung oder eine heterozyklische aromatische Verbindung ist, wobei das Monomer optional Anilin oder ein Derivat davon oder Pyrrol oder Thiophen oder ein Derivat davon wie beispielsweise 3,4-Ethylendioxythiophen (EDOT) ist.

4. Verfahren nach einem der vorhergehenden Ansprüche, wobei die Oberfläche ein elektrisch leitendes Material, optional ein Metall umfasst, wobei das Metall Eisen, Stahl, Messing, Bronze, Zink, Gold, Silber, Platin, Zinn, Kupfer, Blei, Nickel, Chrom, Titan und/oder Aluminium oder eine Legierung davon umfasst, wobei die Oberfläche optional mindestens einen Teil einer Waffe, eines Gebäudes, eines Fahrzeugs, eines Möbels, eines Werkzeugs, eines Schmuckstücks, eines elektrischen Geräts, eines elektronischen Geräts, eines Hammers, eines Schraubendrehers, eines Stemmeisens, einer Schusswaffe, einer Patrone, eines Gehäuses, eines Projektils, einer Hülse, eines Schlüssels, eines Schlosses, eines Fensters, einer Tür, eines Messers, eines Schwerts, eines Bajonetts, einer Machete, eines Griffs, eines Sitzes oder eines Tisches bildet.

5. Verfahren nach einem der vorhergehenden Ansprüche, wobei die Konzentration des Monomers in dem monomerhaltigen Fluid etwa 0,001 M bis etwa 1 M beträgt, wobei das monomerhaltige Fluid einen pH-Wert zwischen 0 und 10 aufweist, vorzugsweise einen pH-Wert von weniger als etwa 3 aufweist, wobei das monomerhaltige Fluid optional eine Säure enthält, die aus Salzsäure, Schwefelsäure, Perchlorsäure und Salpetersäure ausgewählt ist, vorzugsweise Schwefelsäure, wobei die Konzentration der Säure in dem monomerhaltigen Fluid optional etwa 0,001 M bis etwa 2 M beträgt.

6. Verfahren nach einem der vorhergehenden Ansprüche, wobei ein elektrisches Potenzial von etwa 0,1 V bis 2 V angelegt wird, um das Polymer an der Oberfläche abzuscheiden, und das Potenzial im zeitlichen Verlauf konstant gehalten oder variiert wird.

7. Verfahren nach einem der vorhergehenden Ansprüche, wobei das monomerhaltige Fluid vor dem Schritt des Anlegens eines elektrischen Potenzials an das Polymer an der Oberfläche, um den Abdruck sichtbar zu machen, entfernt wird, wobei optional ein Fluid, das im Wesentlichen frei von dem Monomer ist, vor dem Schritt des Anlegens eines elektrischen Potenzials an das Polymer an der Oberfläche, um den Abdruck sichtbar zu machen, mit der Oberfläche in Kontakt gebracht wird, wobei das Fluid, das im Wesentlichen frei von dem Monomer ist, optional einen pH-Wert von etwa 0 bis 10 aufweist, vorzugsweise einen pH-Wert von weniger als etwa 3, wobei das Fluid, das im Wesentlichen frei von dem Monomer ist, optional eine Säure enthält, die aus Salzsäure, Schwefelsäure, Perchlorsäure und Salpetersäure ausgewählt ist, vorzugsweise Schwefelsäure, wobei die Konzentration der Säure in dem Fluid, das im Wesentlichen frei von dem Monomer ist, optional etwa 0,001 M bis etwa 2 M beträgt.

8. Verfahren nach einem der vorhergehenden Ansprüche, wobei der Schritt des Anlegens eines elektrischen Potenzials an die Oberfläche zu einer Änderung der Farbe des Polymerfilms führt, wobei die Farbe des Films optional gelb, grün, rot oder blau ist.

9. Verfahren nach einem der vorhergehenden Ansprüche, wobei ein elektrisches Potenzial von etwa -1 V bis etwa 2 V an die Oberfläche angelegt wird, um den Abdruck sichtbar zu machen, wobei das elektrische Potenzial, das an die Oberfläche angelegt wird, um den Abdruck sichtbar zu machen, optional im zeitlichen Verlauf variiert wird, wobei die Oberfläche optional die Arbeitselektrode in einer Drei-Elektroden-Zelle bildet, wobei die Dicke des Polymers auf der Oberfläche optional etwa 20 nm bis etwa 3 µm beträgt, vorzugsweise etwa 100 nm bis etwa 2,5 µm, stärker bevorzugt etwa 140 nm bis etwa 2 µm und am stärksten bevorzugt etwa 300 nm bis etwa 800 nm.

10. Verfahren nach einem der vorhergehenden Ansprüche, ferner die folgenden Schritte umfassend:
- Aufzeichnen eines Bildes des sichtbar gemachten Abdrucks,
- Analysieren des sichtbar gemachten Abdrucks,
- Vergleichen des sichtbar gemachten Abdrucks mit einem anderen Abdruck,
- Überführen des Abdrucks von einer nichtleitenden Oberfläche auf eine leitende Oberfläche, vorzugsweise unter Verwendung eines geeigneten Überführungsmittels, und
- Entfernen des Polymers von der Oberfläche.

## Revendications

1. Procédé de visualisation d'une empreinte sur une surface, comprenant les étapes consistant à :
- utiliser une surface qui comprend une empreinte,
- mettre en contact la surface avec un fluide contenant un monomère,
- passer un courant électrique entre la surface et le fluide afin de déposer un polymère électrochromique sur la surface, et
- appliquer un potentiel électrique sur la surface afin de visualiser l'empreinte.

2. Procédé selon la revendication 1, dans lequel l'empreinte est une empreinte digitale, une empreinte de l'oreille, une empreinte labiale, une empreinte palmaire, une empreinte plantaire ou une empreinte de chaussure.

3. Procédé selon l'une quelconque des revendications 1 ou 2, dans lequel le monomère est un composé aromatique ou un composé hétérocyclique aromatique, où le monomère est facultativement de l'aniline ou un dérivé de celle-ci, ou du pyrrole ou du thiophène, ou un dérivé de ceux-ci, tel que le 3,4-éthylènedioxythiophène (EDOT).

4. Procédé selon l'une quelconque des revendications précédentes, dans lequel la surface comprend un matériau électriquement conducteur, facultativement un métal, le métal étant du fer, de l'acier, du laiton, du bronze, du zinc, de l'or, de l'argent, du platine, de l'étain, du cuivre, du plomb, du nickel, du chrome, du titane, et/ou de l'aluminium, ou un alliage de ceux-ci, où la surface forme facultativement au moins une partie d'une arme, d'une construction, d'un véhicule, d'un meuble, d'un outil, d'un bijou, d'un dispositif électrique, d'un dispositif électronique, d'un marteau, d'un tournevis, d'une pince à levier, d'une arme à feu, d'une cartouche, d'une douille, d'une balle, d'un obus, d'une clé, d'un verrou, d'une fenêtre, d'une porte, d'un couteau, d'une épée, d'une baïonnette, d'une machette, d'une poignée, d'un siège ou d'une table.

5. Procédé selon l'une quelconque des revendications précédentes, dans lequel la concentration du monomère dans le fluide contenant un monomère est comprise entre environ 0,001 M et environ 1 M, le fluide contenant un monomère ayant un pH compris entre 0 et 10, de préférence un pH inférieur à environ 3, le fluide contenant un monomère comprenant facultativement un acide choisi parmi l'acide chlorhydrique, l'acide sulfurique, l'acide perchlorique et l'acide nitrique, de préférence l'acide sulfurique, la concentration de l'acide dans le fluide contenant un monomère étant facultativement comprise entre environ 0,001 M et environ 2 M.

6. Procédé selon l'une quelconque des revendications précédentes, dans lequel un potentiel électrique d'environ 0,1 V à 2 V est appliqué pour déposer le polymère sur la surface, et le potentiel est maintenu constant ou variable dans le temps.

7. Procédé selon l'une quelconque des revendications précédentes, dans lequel le fluide contenant un monomère est éliminé de la surface avant l'étape d'application d'un potentiel électrique au polymère sur la surface pour visualiser l'empreinte, le fluide qui est sensiblement exempt de monomère étant facultativement mis en contact avec la surface avant l'étape d'application d'un potentiel électrique au polymère sur la surface pour visualiser l'empreinte, le fluide qui est sensiblement exempt de monomère ayant facultativement un pH d'environ 0 à 10, de préférence inférieur à environ pH 3, le fluide qui est sensiblement exempt de monomère comprenant facultativement un acide choisi parmi l'acide chlorhydrique, l'acide sulfurique, l'acide perchlorique et l'acide nitrique, de préférence l'acide sulfurique, la concentration de l'acide dans le fluide qui est sensiblement exempt de monomère étant facultativement comprise entre environ 0,001 M et environ 2 M.

8. Procédé selon l'une quelconque des revendications précédentes, dans lequel l'étape d'application d'un potentiel électrique à la surface entraîne un changement de couleur du film polymère, la couleur du film polymère film étant facultativement jaune, verte, rouge ou bleue.

9. Procédé selon l'une quelconque des revendications précédentes, dans lequel un potentiel électrique compris entre environ -1 V et environ 2 V est appliqué à la surface pour visualiser l'empreinte, où facultativement le potentiel électrique appliqué à la surface pour visualiser l'empreinte est variable dans le temps, où facultativement la surface forme l'électrode de travail d'une cellule à trois électrodes, où facultativement l'épaisseur du polymère sur la surface est comprise entre environ 20 nm et environ 3 µm, de préférence entre environ 100 nm et environ 2,5 µm, plus préférentiellement entre environ 140 nm et environ 2 µm, et idéalement entre environ 300 nm et environ 800 nm.

10. Procédé selon l'une quelconque des revendications précédentes, comprenant en outre les étapes consistant à :
- enregistrer une image de l'empreinte visualisée,
- analyser l'empreinte visualisée,
- comparer l'empreinte visualisée avec une autre empreinte,
- transférer l'empreinte d'une surface non conductrice vers une surface conductrice, de préférence à l'aide d'un moyen de transfert approprié, et
- éliminer le polymère de la surface.
